# EUROPEAN PATENT APPLICATION

(11) **EP 4 129 413 A1**
(43) Date of publication of application: **08.02.2023**
(21) Application number: 21774424.2
(22) Date of filing: 19.03.2021
(51) Int. Cl.: A61Q 11/00, A61K 8/34, A61K 8/49, A61K 8/63, A61K 8/67, A61K 8/73

(54) **COMPOSITION FOR ORAL CAVITY**

(30) Priority: 27.03.2020 JP 2020059152
(71) Applicant: NOF Corporation, Shibuya-ku Tokyo 150-6019 (JP)
(72) Inventor: HARA, Masao, Kawasaki-shi, Kanagawa 210-0865 (JP); SAKURAI, Shunsuke, Kawasaki-shi, Kanagawa 210-0865 (JP)
(74) Representative: J A Kemp LLP
(86) International application number: PCT/JP2021/011299
(87) International publication number: WO 2021/193398

(57) **Abstract**

According to the present invention, a composition for oral cavity that can prevent the formation and maturation of biofilms in the oral cavity, improve the taste and the remaining sensation after use in the oral cavity, and is superior in the sense of use can be provided.

The composition for oral cavity of the present invention contains 0.001% by mass to 10% by mass of proteoglycan, an algefacient, and one or more members selected from the group consisting of an antimicrobial agent, an anti-inflammatory agent, and a vitamin.

## Description

### [Technical Field]

The present invention relates to a composition for oral cavity that can prevent formation and maturation of a biofilm in the oral cavity, and is superior in the sense of use.

### [Background Art]

In general, the oral environment refers to the condition of the entire oral cavity including not only the space in the mouth but also teeth, gum, nasal cavity, palate, oral mucosa, and the like. If the oral environment deteriorates, it causes bad breath, caries, periodontal disease, and the like, leading to decreased quality of life.

Due to the deterioration of the oral environment, ions in liquids and the air, food debris, and the like adsorb onto the tooth surface, forming an adsorption layer called a conditioning film, on which bacteria adhere to form dental plaque (accumulation of bacteria). The adhered bacteria secrete extracellular polysaccharides, forming a colony (aggregates of bacteria), which matures over time, leading to the formation of a biofilm (biological slime, aggregates of multiple bacteria). Dental plaque is relatively easy to remove by oral cleaning such as tooth brushing and the like, but biofilm is difficult to remove by normal oral cleaning such as tooth brushing and the like.

Pathogenic bacteria living in biofilm that cause dental caries and periodontal disease adhere to heart valves and the like and proliferate, causing bacterial endocarditis. Particularly in the elderly people, they can enter the blood flow and cause circulatory disorders such as coronary artery disease, heart disease, and myocardial infarction.

Therefore, in order to maintain a good oral environment and prevent the formation and maturation of biofilms in the oral cavity, antimicrobial agents, anti-inflammatory agents, vitamins, and the like are used. However, since these have peculiar tastes, when blended in compositions for oral cavity, the sense of use is deteriorated. Also, algefacients such as l-menthol and the like are used in compositions for oral cavity to impart a cooling sensation and enhance the sense of use. Particularly in compositions containing cationic antimicrobial agents, however, bitter tastes are problematically enhanced. To alleviate the bitterness in such cases, it has been proposed to use chlorobutanol and anethole in addition to cationic antimicrobial agents and l-menthol (Patent Literature 1).

However, there is a problem that repeated use of a composition for oral cavity with an enhanced cooling sensation leaves a strong remaining sensation in the oral cavity.

Therefore, it has been difficult to achieve both maintenance of a good oral environment and prevention of the formation and maturation of biofilms in the oral cavity, and provision of a composition for oral cavity superior in the sense of use.

### [Citation List]

### [Patent Literature]

[PTL 1]
JP-A-2012-077032

### [Summary of Invention]

### [Technical Problem]

Therefore, the problem of the present invention is to provide a composition for oral cavity that can prevent the formation and maturation of biofilms in the oral cavity, improve the taste and the remaining sensation in the oral cavity, and is superior in the sense of use.

### [Solution to Problem]

The present inventors have conducted intensive studies in an attempt to solve the aforementioned problem and found that the above-mentioned problem can be solved by using, in a composition for oral cavity, proteoglycan, an algefacient, and one or more members selected from the group consisting of an antimicrobial agent, an anti-inflammatory agent, and a vitamin in combination, which resulted in the completion of the present invention.

That is, the present invention relates to the following.
[1] A composition for oral cavity, comprising 0.001% by mass to 10% by mass of proteoglycan, an algefacient, and one or more members selected from the group consisting of an antimicrobial agent, an anti-inflammatory agent, and a vitamin.
[2] The composition of [1], wherein the proteoglycan is a plant proteoglycan.
[3] The composition of [1] or [2], further comprising 0.001% by mass to 10% by mass of a copolymer comprising a constitutional unit based on 2-(meth)acryloyloxyethylphosphoryl choline.
[4] A method for preventing formation and maturation of a biofilm in oral cavity, comprising having a subject in need of prevention of the formation and maturation of a biofilm in the oral cavity use a composition for oral cavity, comprising 0.001% by mass to 10% by mass of proteoglycan, an algefacient, and one or more members selected from the group consisting of an antimicrobial agent, an anti-inflammatory agent, and a vitamin.
[5] The method of [4], wherein the proteoglycan is a plant proteoglycan.
[6] The method of [4] or [5], wherein the composition further comprises 0.001% by mass to 10% by mass of a copolymer comprising a constitutional unit based on 2-(meth)acryloyloxyethylphosphoryl choline.
[7] A method for improving a taste and remaining sensation in oral cavity of a composition for oral cavity, comprising adding 0.001% by mass to 10% by mass of proteoglycan, an algefacient, and one or more members selected from the group consisting of an antimicrobial agent, an anti-inflammatory agent, and a vitamin, to the composition for oral cavity.
[8] The method of [7], wherein the proteoglycan is a plant proteoglycan.
[9] The method of [7] or [8], further comprising adding 0.001% by mass to 10% by mass of a copolymer comprising a constitutional unit based on 2-(meth)acryloyloxyethylphosphoryl choline.

### [Advantageous Effects of Invention]

According to the present invention, a composition for oral cavity that can satisfactorily prevent the formation and maturation of biofilms in the oral cavity, improve the taste and the remaining sensation in the oral cavity, and is superior in the sense of use can be provided.

The composition for oral cavity of the present invention can maintain a good oral environment, and is useful for the prophylaxis of caries and periodontal disease.

In addition, according to the present invention, a method for preventing the formation and maturation of a biofilm in the oral cavity in a subject in need of prevention of the formation and maturation of a biofilm in the oral cavity can be provided.

The above-mentioned method of the present invention is useful in maintaining a good oral environment of the above-mentioned subject, and prevent caries and periodontal disease.

Furthermore, according to the present invention, a method for improving the taste and remaining sensation in the oral cavity of a composition for oral cavity can be provided.

The above-mentioned methods of the present invention are superior in the effect of preventing caries and periodontal disease, and are useful for providing a composition for oral cavity which is superior in the sense of use.

### [Description of Embodiments]

The present invention is further described in detail below.

The present invention provides a composition for oral cavity.

The composition for oral cavity of the present invention contains proteoglycan, an algefacient, and one or more members selected from the group consisting of an antimicrobial agent, an anti-inflammatory agent, and a vitamin.

The proteoglycan contained in the composition for oral cavity of the present invention is a complex of a sugar having a special structure and a protein, and includes animal-derived and plant-derived proteoglycans.

Animal-derived proteoglycans are glycoproteins in which multiple glycosaminoglycans such as chondroitin sulfate, keratan sulfate, and the like are covalently bound to one core protein, and are widely distributed in the body in the organ, brain, skin, cartilage, and the like, as one component of an extracellular matrix.

In plant-derived proteoglycans, arabinogalactan and core protein are bound in a certain manner. They are formally called arabinogalactan-protein (AGP), and are present as an extracellular matrix in the cell walls and sap of plants.

In the present invention, both animal proteoglycans and plant proteoglycans can be used as proteoglycans. In consideration of production costs, the recent tendency to prefer plant-derived starting materials, and the like, plant proteoglycans are preferably used.

As the plant proteoglycan, a plant proteoglycan made from gum arabic obtained from Acacia arabica (Acacia senegal Willdenow), which belongs to Acacia (Mimosoideae, Fabaceae), or related plants of the same genus as a starting material is preferably used. Here, gum arabic is obtained by drying the exudate exuding from the wound of the bark of the aforementioned plant.

Since a plant proteoglycan having a physiological activity equal to or higher than that of animal proteoglycans can be obtained from gum arabic obtained from Acacia arabica (Acacia senegal Willdenow), which belongs to Acacia (Mimosoideae, Fabaceae), or its seyal species, Acacia seyal Delile as a starting material, the plant proteoglycan made from the gum arabic are preferably used for the purpose of the present invention. A plant proteoglycan made from gum arabic obtained from Acacia arabica (Acacia senegal Willdenow) as a starting material is further preferably used.

In the present invention, the plant proteoglycan preferably used as the proteoglycan is obtained as a purified product obtained by removing mainly arabinogalactan and glycoprotein from gum arabic obtained from the above-mentioned plants such as Acacia arabica.

For the purpose of the present invention, plant proteoglycans to be used preferably have a weight average molecular weight of 900,000 to 3,500,000, more preferably 1,000,000 to 3,000,000, as measured by size-exclusion chromatography with online connection of a multi-angle light scattering detector and differential refractive index detector.

In addition, those having a total aldehyde content of 0.005 µmolar equivalents/g to 2 µmolar equivalents/g as measured using an aldehyde quantitation kit such as Amplite (trade mark) aldehyde quantitation kit (colorimetric) (Colorimetric Aldehyde Quantitation Kit) (product number: 10051) (manufactured by AAT Bioquest) and the like are preferably used.

In the present invention, plant proteoglycans may be used in either a liquid form such as aqueous solution, suspension, dispersion, and the like, or a solid form such as powder, granule, and the like.

Only one kind of the above-mentioned proteoglycan can be used or two or more kinds thereof can also be used in combination in the composition for oral cavity of the present invention.

Animal proteoglycans produced by extraction, purification, and the like from animal tissues such as salmon nasal cartilage, shark fin cartilage, squid head cartilage, and the like may be used, or commercially available products provided by various companies may also be used.

In the present invention, animal proteoglycans may be used in either a liquid form such as aqueous solution, suspension, dispersion, and the like, or a solid form such as powder, granule, and the like.

On the other hand, plant proteoglycan is preferably produced by subjecting an aqueous solution of gum arabic adjusted to 0.5% by mass to 40% by mass to purification using a strongly basic porous type I anion exchange resin and a strongly acidic cation exchange resin. It is also possible to use a commercially available product, which is generally available as an aqueous solution having an active ingredient content of about 1% by mass.

Examples of such commercially available plant proteoglycan product include "PHYTOPROTEOGLYCAN (registered trade mark)" manufactured by NOF CORPORATION.

The content of proteoglycan in the composition for oral cavity of the present invention is 0.001% by mass to 10% by mass and, from the aspect of the effect of improving the remaining sensation in the oral cavity, it is preferably 0.005% by mass to 5% by mass.

When the content of proteoglycan is less than 0.001% by mass, a sufficient improving effect on the remaining sensation in the oral cavity cannot be imparted, and when it exceeds 10% by mass, an effect comparable to the content may not be achieved in some cases.

As the algefacient contained in the composition for oral cavity of the present invention, any algefacient can be used without particular limitation as long as it is acceptable for use for oral cavity. Examples thereof include fennel oil, d-camphor, dl-camphor, cinnamon oil, geraniol, methyl salicylate, spilanthol, spearmint oil, thymol, mentha water, mentha oil, peppermint oil, bergamot oil, d-borneol, l-menthol, borneol, and the like.

Only one kind of the above-mentioned algefacient can be used or two or more kinds thereof can also be used in combination.

From the aspects of a cooling sensation-imparting effect and a reducing effect on the bitter taste of the belowmentioned antimicrobial agent and the like, l-menthol, mentha oil, spearmint oil, peppermint oil, and the like are preferably used, and l-menthol and the like are more preferably used as the algefacient.

The content of the algefacient in the composition for oral cavity of the present invention is preferably 0.0001% by mass to 5% by mass, more preferably 0.001% by mass to 1% by mass.

When the content of the algefacient is within the above-mentioned range, a good cooling sensation can be imparted to a composition for oral cavity, and a bitter taste caused by an antimicrobial agent and the like can be reduced.

The composition for oral cavity of the present invention contains one or more members selected from the group consisting of an antimicrobial agent, an anti-inflammatory agent, and a vitamin as an active ingredient for preventing the formation and maturation of a biofilm in the oral cavity.

As the antimicrobial agent contained in the composition for oral cavity of the present invention, any antimicrobial agent can be used without particular limitation as long as it is acceptable for use for oral cavity. Examples thereof include cationic surfactants such as cetylpyridinium chloride and hydrate thereof, dequalinium chloride, benzalkonium chloride, benzethonium chloride, hexadecyltrimethylammonium chloride, and the like; amphoteric surfactants such as alkyldiaminoethylglycine hydrochloride and the like; chlorhexidine salts such as chlorhexidine hydrochloride, chlorhexidine gluconate, and the like; iodine agents such as povidone iodo and the like; phenol derivatives such as isopropylmethylphenol and the like; aromatic ether derivatives such as triclosan and the like, and the like.

Only one kind of the above-mentioned antimicrobial agent can be used or two or more kinds thereof can also be used in combination.

From the aspects of a preventive effect on the formation and maturation of a biofilm in the oral cavity, low stimulation in the oral cavity, and the like, chlorhexidine gluconate, cetylpyridinium chloride and hydrate thereof, benzalkonium chloride, benzethonium chloride, isopropylmethylphenol, and the like are preferably used, and chlorhexidine gluconate, cetylpyridinium chloride and hydrate thereof, and the like are more preferably used as the antimicrobial agent.

As the anti-inflammatory agent contained in the composition for oral cavity of the present invention, any anti-inflammatory agent can be used without particular limitation as long as it is acceptable for oral cavity. Examples thereof include azulene derivatives such as guaiazulene (1,4-dimethyl-7-isopropylazulene), sodium azulenesulfonate, and the like; allantoin and derivatives thereof such as allantoin, allantoin sodium pyrrolidonecarboxylate, allantoin glycyrrhetinic acid, allantoin pantothenyl alcohol, and the like; aloin and a derivatives thereof; glycyrrhizin acid and salts thereof such as dipotassium glycyrrhizinate, monoammonium glycyrrhizinate, and the like, glycyrrhetinic acid and derivative thereof such as β-glycyrrhetinic acid, stearyl glycyrrhetinate, and the like; tropolone derivatives such as hinokitiol and the like; synthetic plasmin,inhibitors such as ε-aminocaproic acid, tranexamic acid, and the like; shikonin and derivatives thereof; 1,8-cineol, and the like.

Only one kind of the above-mentioned anti-inflammatory agent can be used or two or more kinds thereof can also be used in combination.

From the aspect of a preventive effect on the formation and maturation of a biofilm in the oral cavity, allantoin, glycyrrhizin acid and salts thereof, hinokitiol, ε-aminocaproic acid, tranexamic acid, and the like are preferably used, and allantoin, dipotassium glycyrrhizinate, hinokitiol, and the like are more preferably used as the anti-inflammatory agent.

As the vitamin contained in the composition for oral cavity of the present invention, any vitamin can be used without particular limitation as long as it is acceptable for oral cavity. Examples thereof include vitamin B₂ and derivatives thereof such as riboflavin, flavin mononucleotide (FMN), flavin-adenine dinucleotide (FAD), riboflavin butyric acid ester, and the like; vitamin B₆, and salts and derivatives thereof such as pyridoxine hydrochloride, pyridoxine dipalmitate, and the like; pantothenic acid and derivatives thereof such as pantothenic acid, D-pantothenyl alcohol, and the like; ascorbic acid, and salts and derivatives thereof such as sodium L-ascorbate, L-ascorbic acid phosphate magnesium, and the like; vitamin E and derivatives thereof such as dl-α-tocopherol, dl-α-tocopherol acetate, dl-α-tocopherol nicotinate, and the like, and the like.

Only one kind of the above-mentioned vitamin can be used or two or more kinds thereof can also be used in combination.

From the aspect of a preventive effect on the formation and maturation of a biofilm in the oral cavity, dl-α-tocopherol acetate, pantothenic acid, sodium L-ascorbate, and the like are preferably used, and acetic acid dl-α-tocopherol, pantothenic acid, and the like are more preferably used as the vitamin.

The content of one or more members selected from the group consisting of an antimicrobial agent, an anti-inflammatory agent, and a vitamin in the composition for oral cavity of the present invention is preferably 0.001% by mass to 5% by mass, more preferably 0.01% by mass to 2% by mass, as a total amount of these.

When the content of one or more members selected from the group consisting of an antimicrobial agent, an anti-inflammatory agent, and a vitamin is within the above-mentioned range, a good preventive effect on the formation and maturation of a biofilm in the oral cavity can be afforded without showing a strong stimulation to the oral cavity.

Furthermore, the composition for oral cavity of the present invention may contain a copolymer containing a constitutional unit based on 2-(meth)acryloyloxyethylphosphoryl choline (MPC) (hereinafter sometimes denoted as "MFC copolymer").

By further containing the MFC copolymer, the remaining sensation-improving effect in the oral cavity after using the composition for oral cavity can be improved.

As the MFC copolymer that can be contained in the composition for oral cavity of the present invention, a copolymer containing a constitutional unit based on MFC, and a constitutional unit based on a hydrophobic monomer can be mentioned. Examples of the constitutional unit based on a hydrophobic monomer include constitutional units based on alkyl (meth)acrylate, (meth)acryl amide derivative, and the like.

The constitutional unit based on MPC is represented by the following formula (1). In the present specification, the "(meth)acryloyl" means "acryloyl or methacryloyl". 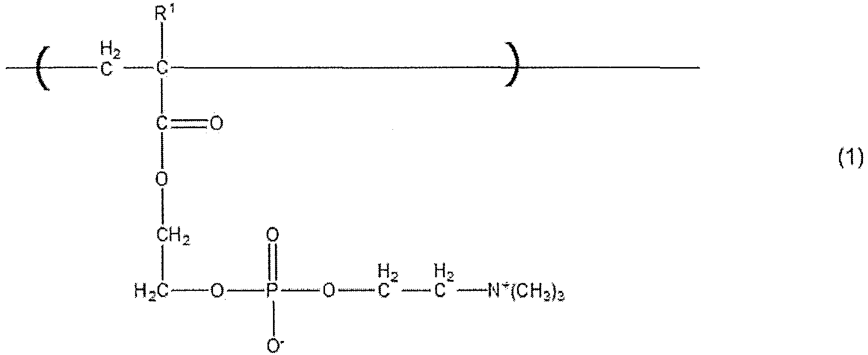 wherein R¹ is a hydrogen atom or a methyl group.

The constitutional unit based on alkyl (meth)acrylate is represented by the following formula (2). As used herein, the "(meth)acrylic acid" means "acrylic acid or methacrylic acid". 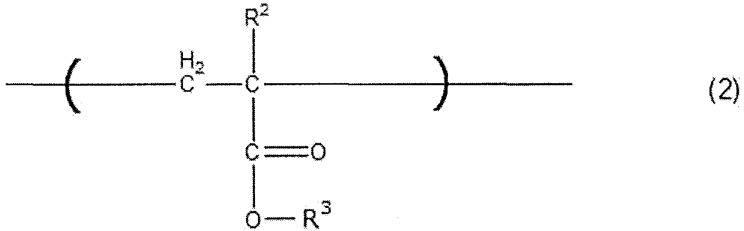 wherein R² is a hydrogen atom or a methyl group, and R³ is an alkyl group having 1 to 24 carbon atoms.

In the formula (2), the alkyl group for R³ is a straight chain or branched alkyl group having 1 to 24 carbon atoms, and examples thereof include methyl, ethyl, propyl, butyl, pentyl, hexyl, octyl, 2-ethylhexyl, decyl, dodecyl, tetradecyl, hexadecyl, octadecyl, docosyl, and the like.

Therefore, as the constitutional unit based on alkyl (meth)acrylate represented by the formula (2), constitutional units based on methyl (meth)acrylate, ethyl (meth)acrylate, propyl (meth)acrylate, butyl (meth)acrylate, 2-ethylhexyl (meth)acrylate, dodecyl(lauryl) (meth)acrylate, tridecyl (meth)acrylate, octadecyl(stearyl) (meth)acrylate, docosyl(behenyl) (meth)acrylate, and the like can be mentioned.

In addition, as the constitutional unit based on alkyl (meth)acrylate, constitutional units based on alkyl (meth)acrylates such as diethylaminoethyl (meth)acrylate, trifluoroethyl (meth)acrylate, heptadecafluorodecyl (meth)acrylate, and the like, in which the alkyl group for R³ in the above-mentioned formula (2) is substituted by a dialkylamino group, a halogen atom, or the like, and 2-hydroxy-3-(meth)acryloyloxypropyltrimethylammonium chloride having a structure in which the alkyl group for R³ is substituted by a hydroxyl group and a quaternary ammonio group are also exemplified as the constitutional unit based on a hydrophobic monomer.

A constitutional unit based on a (meth)acryl amide derivative is represented by the following formula (3). As used herein, the "(meth)acryl amide" means "acrylamide or methacrylamide". 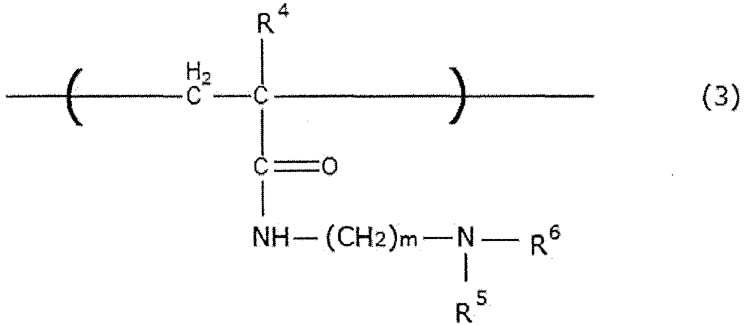 wherein R⁴ is a hydrogen atom or a methyl group, R⁵ and R⁶ are each independently a methyl group or an ethyl group, and m is an integer of 1 to 3.

For the purpose of the present invention, a copolymer containing a constitutional unit based on N,N-dimethylaminoethylacrylamide or N,N-dimethylaminopropylacrylamide is preferred as the constitutional unit based on a (meth)acryl amide derivative.

In the present invention, a copolymer containing a constitutional unit based on MFC, and one or more constitutional units based on the above-mentioned hydrophobic monomers can be used as the MPC copolymer.

For the purpose of the present invention, a copolymer containing one or more members selected from the group consisting of a constitutional unit based on alkyl (meth)acrylate, a constitutional unit based on 2-hydroxy-3-(meth)acryloyloxypropyltrimethylammonium chloride, and a constitutional unit based on a (meth)acryl amide derivative as a constitutional unit based on a hydrophobic monomer is preferably used.

The content ratio of a constitutional unit based on MPC and a constitutional unit based on the above-mentioned hydrophobic monomer ([constitutional unit based on MPC]:[constitutional unit based on hydrophobic monomer]) (n₁:n₂) in the MPC copolymer is 9:1 to 2:1, preferably 8:1 to 7:3, more preferably 6:1 to 3:1, in a molar ratio.

The weight average molecular weight of the MPC copolymer contained in the composition for oral cavity of the present invention is 10,000 to 5,000,000, preferably 20,000 to 1,000,000.

When the weight average molecular weight of the MPC copolymer is less than 10,000, the remaining sensation in the oral cavity may not be improved. On the other hand, when the weight average molecular weight of the MPC copolymer is higher than 5,000,000, the viscosity of the MPC copolymer may increase rapidly, which can make it difficult to prepare the composition for oral cavity.

The weight average molecular weight of the MPC copolymer is measured by gel permeation chromatography (GPC), and indicated by pullulan-equivalent molecular weight.

The polymerization form of the MPC copolymer is not particularly limited, and may be a random copolymer or a block copolymer, with preference given to a random copolymer.

The MPC copolymer may be a copolymer containing other constitutional unit in addition to a constitutional unit based on MPC and a constitutional unit based on a hydrophobic monomer.

Such other constitutional unit can be appropriately selected from those that can generally be constitutional units of copolymers, as long as the effects of the present invention are not affected. Examples of other constitutional unit include constitutional units based on alkenyl (meth)acrylates such as allyl (meth)acrylate and the like; cyclic alkyl (meth)acrylates such as cyclohexyl (meth)acrylate and the like; (meth)acrylates having an alkyl group substituted by an heterocycle such as tetrahydrofurfuryl (meth)acrylate and the like; (meth)acrylates having an aromatic group such as benzyl (meth)acrylate, phenoxyethyl (meth)acrylate, and the like; esters of (meth)acrylic acid and monoterpene alcohol such as isobornyl (meth)acrylate and the like; glycidyl (meth)acrylate; styrene monomers such as styrene, methylstyrene, chloromethylstyrene, and the like; vinyl ether monomers such as methyl vinyl ether, butyl vinyl ether, and the like; vinyl ester monomers such as vinyl acetate, vinyl propionate, and the like, and the like.

One or more from such other constitutional units can be contained, and the content thereof in an MPC copolymer is 40 mol% or less, preferably 20 mol% or less, with respect to the total amount of a constitutional unit based on MPC and a constitutional unit based on a hydrophobic monomer.

The MPC copolymer can be produced by a production method known per se.

For example, it can be produced by polymerizing a monomer mixture containing MPCs, hydrophobic monomers such as alkyl (meth)acrylate and the like, and monomers corresponding to the above-mentioned other constitutional unit as necessary in the presence of a radical polymerization initiator under an inert gas atmosphere of nitrogen and the like, according to a known method such as solution polymerization and the like.

The content ratio of each monomer at that time may be a ratio corresponding to the content ratio of each structural unit in the MPC copolymer.

An MPC copolymer produced by the above-mentioned polymerization method can be used, and products that are commercially available as solutions or dispersions of water or polyhydric alcohol can also be used.

In the composition for oral cavity of the present invention, one kind of the above-mentioned MPC copolymer may be selected and used alone, or two or more kinds thereof may be selected and used in combination.

The form of the MPC copolymer used in the composition for oral cavity of the present invention may be any of solid forms such as powder, granule, and the like, and liquid forms such as solution, suspension, dispersion and the like.

The content of the MPC copolymer in the composition for oral cavity of the present invention can be appropriately selected from, 0.001% by mass to 10% by mass, and preferably selected from 0.005% by mass to 5% by mass, from the aspect of the remaining sensation-improving effect in the oral cavity.

When the content of the MPC copolymer is less than 0.001% by mass, the remaining sensation in the oral cavity may not be improved. On the other hand, when the content of the MPC copolymer exceeds 10% by mass, the remaining sensation in the oral cavity after using the composition for oral cavity of the present invention may increase.

Furthermore, the composition for oral cavity of the present invention may contain medicaments generally used in compositions for oral cavity and other than the above-mentioned antimicrobial agents, anti-inflammatory agents, and vitamins, or additives generally used in compositions for oral cavity and other than algefacients, can also be added as necessary as long as the feature of the present invention is not impaired.

Examples of the above-mentioned medicaments generally used in compositions for oral cavity and other than the above-mentioned antimicrobial agents, anti-inflammatory agents, and vitamins, include hemostasis agent, astringent, cell activator, blood circulation promoter, dentin-strengthening agent, tartardeposition inhibitor, hypersensitivity-improving agent, plaque remover, pigmentation inhibitor, bad breath remover, adsorbent, and the like.

Examples of the additives generally used in compositions for oral cavity and other than algefacients include buffering agent, wetting agent, surfactant, preservative, binding agent, abrasive, thickening agent, solvent, organic acid, inorganic salts, antioxidant, stabilizer, sequestering agent, flavor, sweetening agent, corrigent, colorant, and the like.

In the composition for oral cavity of the present invention, the medicaments generally used in compositions for oral cavity and other than the above-mentioned antimicrobial agents, anti-inflammatory agents, and vitamins, and the additives generally used in compositions for oral cavity and other than algefacients can be each used in amounts generally contained in compositions for oral cavity.

When the composition for oral cavity of the present invention is provided in the semi-solid or liquid form described later, a solvent is generally used.

The solvent is not particularly limited, and purified water and ethanol can be mentioned. In the composition for oral cavity of the present invention, ethanol is particularly preferably used as the solvent at a content of 5% by mass to 20% by mass.

The composition for oral cavity of the present invention can be prepared by adding as necessary the above-mentioned medicaments and additives generally used for compositions for oral cavity to proteoglycan, an algefacient, one or more members selected from the group consisting of an antimicrobial agent, an anti-inflammatory agent, and a vitamin, and further, an MPC copolymer and formulating same into a solid form such as powder, granule, and the like, a semi-solid form such as gel, paste, cream, and the like, or a liquid form such as solution, suspension, dispersion, emulsion, and the like, according to well-known formulation methods, such as the methods described in the Japanese Pharmacopoeia, seventeenth Edition, General Rules for Preparation, [3] Monographs for Preparations.

Therefore, the composition for oral cavity of the present invention can be provided as powdered toothpaste, toothpaste, liquid toothpaste, mouthwash, gargle, oral freshener, dental gel, dental cream, oral spray, and the like. Among these, toothpaste, liquid toothpaste, mouthwash, gargle, and the like are preferred.

The method of using the composition for oral cavity of the present invention is not particularly limited. For example, in the case of a liquid composition for oral cavity, 1 mL to 25 mL thereof is held in the mouth for 30 seconds or more each time to rinse the mouth, generally 1 to 10 times, preferably 1 to 8 times, more preferably 1 to 6 times, further preferably 1 to 4 times, further more preferably 1 to 3 times (particularly preferably in the morning, noon, and evening), per day.

The subject that uses the composition for oral cavity of the present invention is not particularly limited, and it is preferably a mammal including a human.

The composition for oral cavity of the present invention can satisfactorily prevent the formation and maturation of biofilms in the oral cavity, improve the taste of the composition for oral cavity and the remaining sensation in the oral cavity when the composition for oral cavity is used, and is superior in the sense of use.

Therefore, the composition for oral cavity of the present invention maintains a good oral environment, and is useful for the prophylaxis of caries and periodontal disease.

The present invention also provides a method for preventing the formation and maturation of a biofilm in the oral cavity in a subject in need of prevention of the formation and maturation of a biofilm in the oral cavity (hereinafter sometimes to be referred to as "prevention method of the present invention" in the present specification).

The prevention method of the present invention includes having a subject in need of prevention of the formation and maturation of a biofilm in the oral cavity use a composition for oral cavity, containing 0.001% by mass to 10% by mass of proteoglycan, an algefacient, and one or more members selected from the group consisting of an antimicrobial agent, an anti-inflammatory agent, and a vitamin.

In the prevention method of the present invention, the "subject in need of prevention of the formation and maturation of a biofilm in the oral cavity" is not particularly limited as long as it is a human or an animal other than human having or at the risk of having a deteriorated oral environment. It preferably refers to human and mammals other than human such as monkey, dog, cat, cow, horse, sheep, goat, rat, mouse, rabbit, and the like, having or at the risk of having a deteriorated oral environment.

In the prevention method of the present invention, the "composition for oral cavity, comprising 0.001% by mass to 10% by mass of proteoglycan, an algefacient, and one or more members selected from the group consisting of an antimicrobial agent, an anti-inflammatory agent, and a vitamin" is as described above in the composition for oral cavity of the present invention.

The composition for oral cavity to be used by the above-mentioned subject can further contain 0.001% by mass to 10% by mass of an MPC copolymer, and where necessary, can also contain medicaments generally used in compositions for oral cavity and other than the above-mentioned antimicrobial agents, anti-inflammatory agents, and vitamins, and additives generally used in compositions for oral cavity and other than algefacients. The MPC copolymer, and the aforementioned medicaments and additives are also as described above in the composition for oral cavity of the present invention.

In addition, in the prevention method of the present invention, a method for having the above-mentioned subject use the above-mentioned composition for oral cavity is also as described above in the composition for oral cavity of the present invention.

The prevention method of the present invention can satisfactorily prevent the formation and maturation of biofilms in the oral cavity of the above-mentioned subject, maintain a good oral environment of the above-mentioned subject, and beneficially prevent caries and periodontal disease in the above-mentioned subject.

The present invention further provides a method for improving the taste and remaining sensation in the oral cavity of a composition for oral cavity (hereinafter sometimes to be referred to as "the improvement method of the present invention" in the present specification).

The improvement method of the present invention includes adding 0.001% by mass to 10% by mass of proteoglycan, an algefacient, and one or more members selected from the group consisting of an antimicrobial agent, an anti-inflammatory agent, and a vitamin, to a composition for oral cavity.

In the improvement method of the present invention, proteoglycan, an algefacient, and one or more members selected from the group consisting of an antimicrobial agent, an anti-inflammatory agent, and a vitamin, to be added to the composition for oral cavity are as described above in the composition for oral cavity of the present invention.

In the improvement method of the present invention, it is preferable to further add 0.001% by mass to 10% by mass of an MPC copolymer to the composition for oral cavity. The MPC copolymer is as described above in the composition for oral cavity of the present invention.

In the improvement method of the present invention, where necessary, medicaments generally used in compositions for oral cavity and other than the above-mentioned antimicrobial agents, anti-inflammatory agents, and vitamins, or additives generally used in compositions for oral cavity and other than algefacients can be further added to the composition for oral cavity. The aforementioned medicaments and additives are also as described above in the composition for oral cavity of the present invention.

In the improvement method of the present invention, the form, the production method, and the like of the composition for oral cavity are also as described above in the composition for oral cavity of the present invention.

According to the improvement method of the present invention, the taste of the composition for oral cavity and the remaining sensation when using the composition for oral cavity can be improved, and a good sense of use of the composition for oral cavity can be achieved.

### [Example]

The present invention is described in more detail in the following by referring to Examples, which are not to be construed as limitative.

### [Examples 1 to 6, Comparative Examples 1 to 6] Composition for oral cavity

According to the formulations shown in Table 1, the respective compositions for oral cavity of Examples 1 to 6 and Comparative Examples 1 to 6 were prepared by the method shown below.

That is, an algefacient (l-menthol) was added to ethanol and dissolved by heating slowly. Purified water (about 80 g) was added, an antimicrobial agent (cetylpyridinium chloride hydrate), an anti-inflammatory agent (dipotassium glycyrrhizinate) or a vitamin (dl-α-tocopherol acetate), plant proteoglycan, or further an MPC copolymer were added, mixed, and stirred to uniformity. Purified water was added to make the total amount 100 g to give the respective compositions for oral cavity of Examples 1 to 6 and Comparative Examples 1 to 6.

In the preparation of the respective compositions for oral cavity of the Examples, "PHYTOPROTEOGLYCAN" (manufactured by NOF CORPORATION) (weight average molecular weight = about 1,200,000, aldehyde content = 1.8 µmolar equivalents/g) was used as plant proteoglycan, and 2-methacryloyloxyethylphosphoryl choline-butyl methacrylate copolymer (copolymerization composition ratio of 2-methacryloyloxyethylphosphoryl choline and butyl methacrylate [2-methacryloyloxyethylphosphoryl choline/butyl methacrylate] (molar ratio) = 80/20, weight average molecular weight = 600,000) was used as MPC copolymer.

In the preparation of the respective compositions for oral cavity of the Examples and Comparative Examples, products commercially available as the starting materials of pharmaceutical products for oral cavity or quasi-drugs for oral cavity were used for ethanol, l-menthol, cetylpyridinium chloride hydrate, dipotassium glycyrrhizinate, and dl-α-tocopherol acetate.

The respective compositions for oral cavity of the Examples 1 to 6 and Comparative Examples 1 to 6 were evaluated for the taste when held in the mouth and the remaining sensation in the oral cavity after use as shown below by sensory evaluation by 20 male and female panelists aged 25 to 55.

### (1) Evaluation of taste

Each panelist was requested to gargle with 10 mL of the respective compositions for oral cavity of the Examples and Comparative Examples for 20 seconds, and evaluate and score the taste sensed at that time according to the following evaluation criteria. The scores of the 20 panelists were totaled, and the unpleasant taste felt in each composition for oral cavity was indicated by "○" or "Δ".

### <Evaluation criteria>

unpleasant taste was not sensed: 2 points
unpleasant taste was sensed somewhat: 1 point
unpleasant taste was sensed: 0 point

### <Judgment criteria>

21 points to 40 points: O; composition for oral cavity without unpleasant taste
20 points or less: Δ; composition for oral cavity with unpleasant taste

### (2) Evaluation of remaining sensation in oral cavity after use

Each panelist was requested to gargle with 10 mL of the respective compositions for oral cavity of the Examples and Comparative Examples for 20 seconds, and then spit it out. After repeating this operation five times, they evaluated and scored the remaining sensation in the oral cavity according to the following evaluation criteria. The scores of the 20 panelists were totaled, and the remaining sensation of each composition in the oral cavity after use was indicated by "⊙", "○" or "Δ" .

### <Evaluation criteria>

remaining sensation in oral cavity was not sensed: 2 points
remaining sensation in oral cavity was sensed somewhat: 1 point
remaining sensation in oral cavity was sensed: 0 point

### <Judgment criteria>

31 points to 40 points: ⊙; composition for oral cavity without remaining sensation in oral cavity
21 points to 30 points: O; composition for oral cavity with rather less remaining sensation in oral cavity 20 points or less: Δ; composition for oral cavity with remaining sensation in oral cavity

The evaluation results are also shown in Table 1.

**[Table 1]**

| composition | | Ex. 1 | Ex. 2 | Ex. 3 | Ex. 4 | Ex. 5 | Ex. 6 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|---|---|---|---|---|---|
| component | ethanol | 10 | 10 | 10 | 10 | 10 | 10 | 10 | 10 | 10 | 10 | 10 | 10 |
| | l-menthol | 0.01 | 0.01 | 0.01 | 0.01 | 0.01 | 0.01 | - | 0.01 | - | 0.01 | - | 0.01 |
| | cetylpyridinium chloride hydrate | 0.01 | 0.01 | 0.01 | 0.01 | - | - | 0.01 | 0.01 | - | - | - | - |
| | dl-α-tocopherol acetate | - | - | - | - | 1 | - | - | - | 1 | 1 | - | - |
| | dipotassium glycyrrhizinate | - | - | - | - | - | 0.2 | - | - | - | - | 0.2 | 0.2 |
| | plant proteoglycan | 0.01 | 0.05 | 0.5 | 0.05 | 0.05 | 0.05 | - | - | - | - | - | - |
| | MPC copolymer | - | - | - | 0.05 | 0.05 | 0.05 | - | - | - | - | - | - |
| | purified water | rest | rest | rest | rest | rest | rest | rest | rest | rest | rest | rest | rest |
| total | | 100 | 100 | 100 | 100 | 100 | 100 | 100 | 100 | 100 | 100 | 100 | 100 |
| evaluation item | taste | ○ | ○ | ○ | ○ | ○ | ○ | Δ | ○ | Δ | ○ | Δ | ○ |
| | remaining sensation | ○ | ○ | ○ | ⊙ | ⊙ | ⊙ | Δ | Δ | Δ | Δ | Δ | Δ |

| | | | | | | | | | | | | | |
|---|---|---|---|---|---|---|---|---|---|---|---|---|---|
| * numerical value in Table shows content (% by mass) of each component ** "-" in Table shows absence of component | | | | | | | | | | | | | |

As shown in Table 1, none of the compositions for oral cavity of Examples 1 to 6 of the present invention had an unpleasant taste, and the remaining sensation in the oral cavity after use was evaluated to be "none" (⊙) or "rather less" (○).

Particularly, it was evaluated that the respective compositions for oral cavity of Examples 4 to 6 containing l-menthol, any of cetylpyridinium chloride hydrate, dl-α-tocopherol acetate, and dipotassium glycyrrhizinate, plant proteoglycan, and an MPC copolymer showed a high improving effect on the remaining sensation in the oral cavity after use, and the remaining sensation in the oral cavity caused by 1-menthol was hardly felt.

In contrast, the compositions for oral cavity of Comparative Examples 1, 3, and 5, which contained an antimicrobial agent, a vitamin or an anti-inflammatory agent but did not contain l-menthol, all had an unpleasant taste.

In all of the compositions for oral cavity of Comparative Examples 2, 4, and 6, which contained l-menthol in addition to an antimicrobial agent, a vitamin or an anti-inflammatory agent, but did not contain plant proteoglycan, the unpleasant taste was improved but the remaining sensation after use was sensed in the oral cavity.

From the above-mentioned results, it was confirmed that the composition for oral cavity of the present invention containing plant proteoglycan, an algefacient, and one or more members selected from the group consisting of an antimicrobial agent, an anti-inflammatory agent, and a vitamin can prevent formation and maturation of a biofilm in the oral cavity, reduces an unpleasant taste caused by an antimicrobial agent and the like, and also improves the remaining sensation in the oral cavity caused by an algefacient.

### [Industrial Applicability]

As described in detail above, according to the present invention, a composition for oral cavity that can satisfactorily prevent the formation and maturation of biofilms in the oral cavity, reduce an unpleasant taste, improve the remaining sensation after use in the oral cavity, and is superior in the sense of use can be provided.

The composition for oral cavity of the present invention maintains a good oral environment, and is useful for the prophylaxis of caries and periodontal disease.

In addition, according to the present invention, a method for preventing the formation and maturation of a biofilm in the oral cavity in a subject in need of prevention of the formation and maturation of a biofilm in the oral cavity can be provided.

The above-mentioned prevention method of the present invention is useful in maintaining a good oral environment of the above-mentioned subject, and prevent caries and periodontal disease.

Furthermore, according to the present invention, a method for improving the taste and remaining sensation in the oral cavity of a composition for oral cavity can be provided.

The above-mentioned improvement method of the present invention is useful for providing a composition for oral cavity which is superior in the sense of use.

This application is based on a patent application No. 2020-059152 filed in Japan, the contents of which are incorporated in full herein.

## Claims

1. A composition for oral cavity, comprising 0.001% by mass to 10% by mass of proteoglycan, an algefacient, and one or more members selected from the group consisting of an antimicrobial agent, an anti-inflammatory agent, and a vitamin.

2. The composition according to claim 1, wherein the proteoglycan is a plant proteoglycan.

3. The composition according to claim 1 or 2, further comprising 0.001% by mass to 10% by mass of a copolymer comprising a constitutional unit based on 2-(meth)acryloyloxyethylphosphoryl choline.
